# EUROPEAN PATENT SPECIFICATION

(11) **EP 2 646 218 B1**
(45) Date of publication and mention of the grant of the patent: **06.04.2016**
(21) Application number: 11728098.2
(22) Date of filing: 26.05.2011
(51) Int. Cl.: B29C 45/00, B29C 45/77, A61M 5/32, A61M 5/158, A61B 5/00, A61B 5/145, A61B 5/15, A61K 9/00, A61M 37/00, B29L 31/00, B29K 105/00

(54) **LIQUID CRYSTALLINE POLYMER MICRONEEDLES**
MIKRONADELN AUS FLÜSSIGKRISTALLINEM POLYMER
MICRO-AIGUILLES DE POLYMÈRE CRISTALLIN LIQUIDE

(30) Priority: 02.12.2010 US 419049 P
(43) Date of publication of application: 09.10.2013
(60) Divisional application: 16154565.2
(73) Proprietor: 3M Innovative Properties Company, Saint Paul, MN 55133-3427 (US)
(72) Inventor: DUAN, Daniel C., Saint Paul, Minnesota 55133-3427 (US); RENDON, Stanley, Saint Paul, Minnesota 55133-3427 (US)
(74) Representative: Vossius & Partner Patentanwälte Rechtsanwälte mbB
(86) International application number: PCT/US2011/038026
(87) International publication number: WO 2012/074576

(56) References cited:
- EP-A1- 0 447 726
- EP-A1- 1 222 937
- WO-A1-00/72901
- WO-A1-90/07348
- WO-A1-2004/009172
- WO-A2-02/34017
- WO-A2-2006/062974
- WO-A2-2010/117602
- DE-A1- 19 856 162
- US-A- 5 151 231
- BRAUN P ET AL: "ORIENTIERUNGEN IN THERMOTROPEN LCP. ÖORIENTATION IN THERMOTROPIC LIQUID CRYSTALLINE POLYMERS (LCPS)", KUNSTOFFE INTERNATIONAL, CARL HANSER VERLAG, MUNCHEN, DE, vol. 84, no. 12, 1 December 1994 (1994-12-01), pages 1705-1707, XP000484733, ISSN: 0023-5563

## Description

The present pertains to liquid crystalline polymer microneedles, particularly to thermotropic liquid crystalline polymer microneedles.

WO 2010/117602 A2 relates to a method of manufacturing a hollow microneedle array. It also relates to the articles therefrom and the use of the articles in applications such as delivering fluid to and/or extracting body fluid from a subject.

WO 02/34017 A2 relates to liquid crystalline polymers. The liquid crystalline polymers may be melt to form very fine features such as ridges and/or channels molded into the resulting solid article. Ridges and/or grooves as little as 0.1 micron wide and/or a little as 10 nm deep and/or ridges or grooves as little as 0.2 micron apart may be molded into LCPs under the proper molding conditions. Other features of similar sizes, such as depressions or high points, may also be molded into LCP parts.

### SUMMARY OF THE INVENTION

In one aspect, the present description provides a device comprising a thermotropic liquid crystalline polymer microneedle.

In another aspect, the present description provides a device comprising an array of liquid crystalline polymer microneedles wherein the microneedles have a depth of penetration of from 50 to 120 microns using 8.89 Newton (2 pounds of force) for 10 seconds.

In another aspect, the present description provides a device comprising an array of liquid crystalline polymer microneedles wherein the microneedles have a depth of penetration of from 50 to 150 microns using 13.34 Newton (3 pounds of force) for10 seconds.

In yet another aspect, the present description provides a device comprising an array of liquid crystalline polymer microneedles wherein the microneedles have a penetration efficiency of 70% or higher using 13.34 Newton (3 pounds of force) for 10 seconds.

In another embodiment, the present description provides a method for making thermotropic liquid crystalline polymer microneedles and microneedle arrays.

### BRIEF DESCRIPTION OF THE DRAWINGS

In the accompanying drawings:

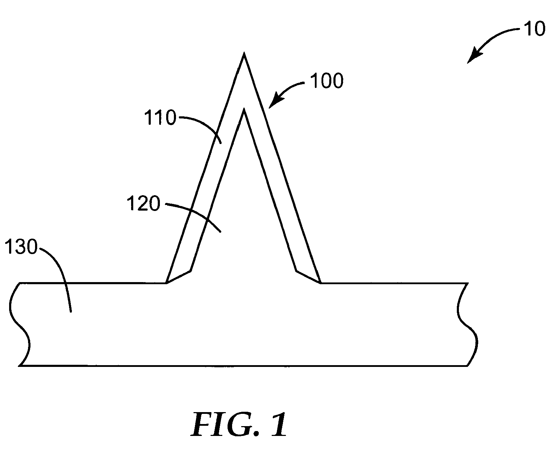

FIG. 1 is a cross sectional side view of a device of the present description;

### DETAILED DESCRIPTION OF CERTAIN EMBODIMENTS

The molding process of the present description can offer one or more of the following advantages: the ability to tailor macroscopic properties of a molded article by selectively controlling the extent to which mesogens in the thermotropic liquid crystalline polymer ("TLCP") flow align and/or flow tumble during molding, the ability to reliably reproduce the shape of the article mold cavity in the resulting molded article, the ability to produce article features of submicrometer dimensions, the ability to produce fine article features having anisotropic physical properties, and/or the ability to produce fine article features having balanced physical mesoscopic properties.

In commercially relevant TLCPs, mesogens constitute the most fundamental unit of the liquid crystal polymer that induces structural order in an arrangement known as a "nematic," which is characterized by mesophase units that display long range orientational order, but only short range packing or positional order. The alignment of the mesogens along an average orientation direction (or vector) defined as the "director" can be characterized by a molecular anisotropy factor (from now on referred to as the "anisotropy factor") which ranges from 0 for a random distribution of mesogen molecular orientations (i.e., isotropic) and 1 for perfect molecular alignment.

As used herein, the term "molten TLCP" refers not only to all of a TLCP being in a molten state (i.e., where its mesogens can flow tumble) but also to a TLCP having its mesogens in the form of solid oriented crystalline regions (e.g., where the mesogens are flow aligned) with the remainder of the TLCP being molten (e.g., being in the form of one or a plurality of fluid amorphous regions).

As used herein, the term "flow aligned" refers to the TLCP mesogens exhibiting an anisotropy factor in the range of from at least about 0.4 up to 1.0, preferably from at least about 0.5 up to but less than 1.0, and more preferably from about 0.6 to less than 1.0, relative to the flow direction.

In accordance with the present invention, the molten composition filling each mold chamber is considered to have a substantial portion of its TLCP mesogens flow aligned, depending on the application, when at least about 30% up to 100%, 35% up to 100%, 40% up to 100%, 45% up to 100%, 50% up to 100%, 55% up to 100%, 60% up to 100%, 65% up to 100%, 70% up to 100%, 75% up to 100%, 80% up to 100%, 85% up to 100%, 90% up to 100%, or 95% up to 100% of the TLCP mesogens filling the mold chamber are flow aligned. Correspondingly, the portion of the device or molded article formed by each mold chamber (e.g., each microneedle) is considered to have a substantial portion of aligned TLCP mesogens, when at least about 30% up to 100%, 35% up to 100%, 40% up to 100%, 45% up to 100%, 50% up to 100%, 55% up to 100%, 60% up to 100%, 65% up to 100%, 70% up to 100%, 75% up to 100%, 80% up to 100%, 85% up to 100%, 90% up to 100%, or 95% up to 100% of the TLCP mesogens across the microneedle are flow aligned. It can be desirable for a maximum of about 25% of the TLCP mesogens across the microneedle to be flow tumbled (i.e., for a maximum of about 25% of the minor dimension of the corresponding portion of the device or molded article to be isotropic). For other applications, higher amounts of flow tumbled TLCP mesogens in each microneedle may be tolerable. For other applications, even a lower maximum of flow tumbled TLCP mesogens microneedle may be desirable.

TLCP mesogens will quickly begin to rotate - from a flow aligned state - at molding temperatures where the TLCP is molten. As a result, the molten TLCP in at least the microneedle molding chamber should be solidified rapidly, after the microneedle molding chamber is filled, to ensure that a substantial amount of the flow aligned mesogens remain flow aligned in the molded microneedle. The dimensions of each microneedle mold cavity can affect the cooling rate of the molten TLCP in the cavity. For example, the smaller the dimension of the mold cavity, the faster the cooling rate, because there is less molten TLCP to be solidified. In addition, if the mold is made of a less thermally conductive material such as, e.g., stainless steel versus a more thermally conductive material such as, e.g., a copper alloy, larger or bulkier mold designs would result in a slower cooling rate, because a mold material having a lower thermal conductivity would conduct heat away from the molten TLCP at a slower rate. This cooling rate will determine whether a suitable amount of the TLCP mesogens will remain flow aligned to provide the physical properties desired in the solidified molded article.

The flow aligned TLCP mesogens can be found throughout the molded element of the article formed by each cavity or at least formed by each microneedle molding cavity. Alternatively, the flow aligned TLCP mesogens of the molded element, formed by each microneedle molding cavity can be found in an outer zone or thickness (e.g., a skin) that encases a core of the molten composition that contains TLCP mesogens that are not flow aligned, as compared to the outer zone or thickness, (e.g., that are mostly or completely flow tumbled or otherwise isotropic). Such an outer zone or thickness of flow aligned TLCP mesogens can be formed under three possible conditions: (1) when the minor dimension is too large to allow for a sufficiently high flow rate across the entire minor dimension, (2) when only the outer portion is cooled fast enough to solidify the mesogens in a flow aligned state, or (3) both (1) and (2). When the microneedle dimension is small enough and the cooling rate of the molten TLCP fast enough, all the mesogens in the molten TLCP across the entire microneedle can be in a flow aligned state and remain flow aligned when the molten TLCP is solidified.

The specifics of the method for making microneedles according to the present description are described in U.S. Provisional Patent Application 61/287,799 (filed December 18, 2009). The following exemplary methods describe the molding procedure associated with injection molding of a TLCP microneedle array using a laminate cavity tool construction.

The method begins by plasticizing solid pellets of neat TLCP material (for example Vectra MT1300, available from Ticona Engineering Polymers, Inc, Florence, KY) using a Krauss-Maffei injection molding machine (available from Krauss-Maffei, Florence, KY) equipped with a 22 mm reciprocating screw designed for low to medium compression. The material is introduced into the feed section of the screw at temperatures near the nominal melting temperature of the polymer (e.g., for Vectra MT1300, < 248.8 °C (480F)) to ensure limited thermal history exposure of the material. As the screw rotates, the material begins to plasticize and move into the compression region of the screw where the polymer begins to exhibit high shear forces which begin the melting process of the softening material up to 271.1 °C (520 F) to 282.2 °C (540 F).

As the injection shot is established, the polymer enters past the metering portion of the screw where it is ready for injection (now at 293.3 °C or 560 F). The material can also be further introduced into a hot manifold (or hot runner) prior to injection into the cavity of interest, The hot runner drops are kept at the same nominal temperature as the metering zone of the screw.

Once the material shot is established and the polymer is in thermal equilibrium with the screw and barrel assembly, the cycle is ready to begin. The TLCP is held at a temperature that reduces the amount of anisotropy gained during the plasticizing process and also ensures the polymer does not prematurely freeze near the point of entry into the mold.

The mold closes and establishes sufficient clamp force between 20 to 30 tons to overcome the internal cavity pressure (greater than 13.7 x 10^7 pascal (20 Kpsi)) when material is injected. Upon clamp force buildup, the TLCP is injected at high speed (> 10.16 cm/s (4 inch/s)) by the ram of the injection unit assuming sufficient first stage pressure (greater than 17.235 x 10^7 pascal (25 Kpsi)) is available to reach the desired velocity. The mold temperature is kept steady at 82°C (180 F) throughout the duration of the cycle.

The entering polymeric material sees extremely high shear rates through the injection gate which promotes a high degree of molecular anisotropy ensuring mechanical properties of the molded microneedles are preferentially in the long axis of the needle (that is, for a square pyramidal needle, along the axis from the base to the tapered tip). It is during this step that the material undergoes a transition from its equilibrium 'flow tumbling' state to one of flow alignment' (see U.S. Provisional Patent Application 61/287,799 (filed December 18, 2009))

Another processing element for TLCP is the use of P20 or 420 stainless steel laminated tooling that helps promote evacuation of air from the cavity concurrently with the speed of injection of the material. The viscosity of air is low enough that it allows the gas to exit via the submicron surfaces that define the needle geometry. Concurrently, the viscosity of the rapidly entering, orienting, and solidifying polymer is thus too high to enter the submicron surfaces defined by the laminated tooling cavity so only air is able to escape.

The first stage fill typically occurs in less than 0.3 seconds depending on the extent of the flow path for the material. More typically 0.1 seconds is needed to completely fill the cavity during the first stage injection.

Once the material enters and has filled about 98% of the cavity using first stage injection (velocity-controlled) the remaining portion of the mold is filled using second stage (pressure-controlled) injection.

Second stage pressure at or close to 12.41 x 10^7 pascal (18 Kpsi) ensures that any remaining portions of the mold (macro- or micro-) are completely filled and that any material shrinkage is compensated by extra material that is applied through the small polymer cushion available in front of the screw.

Upon rapid solidification of the material below its heat distortion temperature (HDT) of 176.6 °C (350 F), the microneedle array(s) is now ready for the ejection phase of the process.

The ejection is the mechanism for removal of the molded part from the injection mold cavity. This typically can happen with a series of ejection pins, air assist, vacuum assist, etc to ensure the part can be adequately removed from the tool.

Comparative examples of injection molded polycarbonate arrays may also be made. The following describes a comparative molding procedure associated with injection molding of a polycarbonate (PC)-based microneedle array using a laminate cavity tool construction.

The method begins by plasticizing solid pellets of neat polycarbonate ("PC") material (Lexan HPS1R, available from Sabic Innovative Plastics, Pittsfield, MA) using a Krauss-Maffei injection molding machine equipped with an 18 mm reciprocating injection molding screw designed for medium compression.

The material is introduced into the feed section of the screw at temperatures near the nominal melting temperature of the polymer (e.g., for Lexan, < 248.8 °C (480F)) to ensure limited thermal history exposure of the material. As the screw rotates, the material begins to plasticize and move into the compression region of the screw where the polymer begins to exhibit high shear forces which begin the melting process of the softening material up to 282.2 °C (540 F).

As the injection shot is established, the polymer enters past the metering portion of the screw where it is ready for injection (still at 282.2 °C (540 F), keeping a linear temperature profile across the length of the screw). The material can also be further introduced into a hot manifold (or hot runner) prior to injection into the cavity of interest. The hot runner drops are kept at the same nominal temperature as the metering zone of the screw.

Once the material shot is established and the polymer is in thermal equilibrium with the screw and barrel assembly, the cycle is ready to begin. In order to ensure that polycarbonate can fully replicate the surface of the laminated mold cavity, the mold temperature needs to be raised to an appropriate state that ensures the entering polymer temperature does not drop below the softening point and has low enough viscosity to fill the cavity of interest.

The mold temperature near the cavity is rapidly increased to a temperature of 165.5 °C (330 F) to 182.2 °C (360 F). This temperature will vary depending on material shear viscosity, melt flow index, macro to micro mold cavity features sizes and venting. This temperature is held long enough to ensure complete fill of the cavity of interest prior to second stage. Once the temperature is reached, the mold closes and applies sufficient clamp force (20 to 30 tons) to overcome the internal cavity pressure (greater than 11. 03 x 10^7 pascal (16 Kpsi)) when material is injected. Upon clamp force buildup, the PC is injected at relatively low speeds (< 5.08 cm (2 inch/s)) by the ram of the injection unit assuming sufficient first stage pressure is available to reach the desired velocity.

The entering material will see high shear forces, however, these forces will be substantially lower relative to traditional PC injection molding given that mold temperature is kept so high during first stage injection. This will ensure that there are minimal thermal and shear stresses associated with the final article after cooling. In turn, this will help promote uniform and balanced macroscopic and microscopic properties in the article.

Useful to the processing of a PC microneedle array using laminated tooling is the ability to evacuate the cavity from air concurrently with the speed of injection of the material. The viscosity of air is low enough that it allows the gas to exit via the submicron surfaces that define the needle geometry. Concurrently, the viscosity of the rapidly entering, orienting, and solidifying polymer is too high to enter the submicron surfaces defined by the laminated tooling cavity.

The mold consists of 196 (14 x 14) cavities each 700 microns deep and converging to a tip of less than 5 microns across. The first stage fill typically occurs in less than 2 seconds depending on the extent of the flow path for the material. More typically 1 second is needed to completely fill the cavity during first stage injection.

Once the material has entered and filled 98% of the cavity using first stage injection (velocity-controlled) the remaining portion of the mold is filled using second stage (pressure-controlled) injection. Second stage injection ensures that any remaining portions of the mold (macro- or micro-) are completely filled and that any material shrinkage is compensated by this extra amount of material that is applied through the small polymer cushion available in front of the screw.

It is during the second stage that the mold temperature (115.5 °C (240 F) to 121. 1 °C (250 F)) is lowered dramatically to bring the polymer temperature below the glass transition of the material. Once the solidification of the material is complete (ensuring the polymer is below its HDT), the microneedle array(s) is now ready for the ejection phase of the process.

The ejection is the mechanism for removal of the molded part from the injection mold cavity. This typically can happen with a series of ejection pins, air assist, vacuum assist, etc to ensure the part can be adequately removed from the tool.

Each of the above described exemplary methods can be used to form a variety of different molded articles. In addition, the filling of each feature cavity with molten composition can result in a structural feature that is hollow (e.g., a hollow needle), a structural feature that is solid (e.g., a solid needle or pin), or a combination of both.

In one aspect, the present description provides a device comprising a thermotropic liquid crystalline polymer microneedle.

As used in this application, microneedles are typically less than 1000 microns in height, less than 800 microns in height, and sometimes less than 500 microns in height. The microneedles may be more than 50 microns in height, more than 200 microns in height, and sometimes more than 500 microns in height.

Microneedles may be characterized by an aspect ratio. As used herein, the term "aspect ratio" is the ratio of the height of the microneedle (above the surface surrounding the base of the microneedle) to the maximum base dimension, that is, the longest straight-line dimension that the base occupies (on the surface occupied by the base of the microneedle). In the case of a pyramidal microneedle with a rectangular base, the maximum base dimension would be the diagonal line connecting opposed corners across the base. Microneedles according to the present description typically have an aspect ratio of between about 2:1 to about 6:1 and sometimes between about 2.5:1 to about 4:1.

The microneedle arrays prepared according to any of the embodiments described herein may comprise any of a variety of configurations, such as those described in the following patents and patent applications, the disclosures of which are herein incorporated by reference. One embodiment for the microneedle devices comprises the structures disclosed in U.S. Patent Application Publication No. 2003/0045837. The disclosed microstructures in the aforementioned patent application are in the form of microneedles having tapered structures that include at least one channel formed in the outside surface of each microneedle. The microneedles may have bases that are elongated in one direction. The channels in microneedles with elongated bases may extend from one of the ends of the elongated bases towards the tips of the microneedles. The channels formed along the sides of the microneedles may optionally be terminated short of the tips of the microneedles. The microneedle arrays may also include conduit structures formed on the surface of the substrate on which the microneedle array is located. The channels in the microneedles may be in fluid communication with the conduit structures. Another embodiment for the microneedle devices comprises the structures disclosed in co-pending U.S. Patent Application Publication No. 2005/0261631 which describes microneedles having a truncated tapered shape and a controlled aspect ratio. Still another embodiment for the microneedle arrays comprises the structures disclosed in U.S. Pat. No. 6,313,612 (Sherman, et al.) which describes tapered structures having a hollow central channel. Still another embodiment for the microneedle arrays comprises the structures disclosed in International Publication No. WO 00/74766 (Gartstein, et al.) which describes hollow microneedles having at least one longitudinal blade at the top surface of tip of the microneedle.

In one embodiment, the microneedle comprises mesogens that are molecularly aligned by an anisotropy factor in the range of from greater than 0.3 up to 1.0. The molding process described herein may lead to microneedles having such anisotropy. For instance, Figure 1 shows a cross-section of a device according to the present description. Device 10 comprises base 130 and microneedle 100. Microneedle 100 further comprises aligned mesogenic layer 110 and unaligned mesogenic layer 120. Base 130 may have layers that are more or less aligned than unaligned mesogenic layer 120.

It should be noted that while in Figure 1 aligned mesogenic layer 110 is shown to terminate at the base, there may be a layer having some degree of alignment along the surfaces of base 130 (both proximate to the microneedle and along the surface opposite the microneedle). More generally, in any location that the resin comes into contact with the mold there may be some degree of mesogenic alignment, particularly where such regions are able to cool rapidly enough to conserve alignment. Such alignment may be due to the flow stress experienced by the TLCP as it fills the mold cavity and accompanying rapid cooling of the TLCP closest to the mold surface.

Devices of the present description may provide microneedles having a high flexural modulus, for instance, allowing for facile penetration of the skin. Further, microneedles of the present description are tough. By "tough" it is meant that devices according to the present description do not experience sheering needle fracture from the base. Particularly, it is observed for devices of the present description that while a microneedle may fracture at its base upon application of a high enough sheer force at its mid-point, the needles tend to not separate from the base.

In some embodiments, the microneedle is integral with and protrudes from base 130. As described in the method above, at least a portion of the mesogens may be flow aligned. In further embodiments, at least about 30% of the mesogens are flow aligned. In yet another embodiment, at least about 10% of the mesogens are flow aligned and the remainder of the mesogens have a relatively isotropic orientation state.

Without wishing to be bound by theory, it is believed that the highly aligned mesogenic "skin" of aligned mesogenic layer 110 may provide the strength to allow for facile skin penetration by devices of the present description. The unaligned mesogentic layer, on the other hand, may, by virtue of its unaligned state as well as the unaligned state of base 130, provide the toughness to allow the microneedles to resist fracture failure typically observed in highly crystalline molded polymer microneedle devices.

In a further embodiment, the device of the present description comprises a microneedle that itself comprises a tip and a base. To illustrate the stiffness of the needle, it may be observed in certain embodiments that the microneedle has a bending moment as measured at 15% of the distance from the tip to the base of from 30,000 to 60,000 mN-µm. In yet other embodiments, the microneedle has a bending moment as measured at 60% of the distance from the tip to the base of from 85,000 to 105,000 mN-µm.

The performance of a device comprising a microneedle may be illustrated by the buckling force required to be applied to the tip of the microneedle before catastrophic deformation is observed. For instance, in one embodiment, the device of the present description comprises a microneedle having a buckling force of from 0.2 to 0.5 N. For reference, as shown in the Examples, similar buckling force values for polycarbonate microneedles is about 0.1 N, less than half the force required to bring about catastrophic deformation in the microneedles of the present description.

Yet another way to describe devices of the present description is to characterize the elastic modulus of a cross section of the microneedle. For instance, in one embodiment, the molecularly aligned mesogen-containing area of the microneedle has an elastic modulus of from 6 to 8 Gpa. In another embodiment, when the microneedle further comprises an area where the mesogens are substantially isotropic (e.g., unaligned mesogenic layer 120) the isotropic area has an elastic modulus of from 4 to 6 Gpa. This differential elastic modulus may allow for the facile insertion of the microneedle into the skin while at the same time reducing the tendency of the microneedle to fracture.

Devices of the present description have microneedles that may be described by their degree of orientation. For instance, the Hermans orientation function is a relatively simple mathematical expression which describes the degree of orientation of a particular polymeric sample. For instance, PC arrays produced as described above shows only diffuse scattering of x-rays and no evidence of aligned crystalline materials. For TLCP microneedles according to the present description, however, Hermans orientation function values of 0.4 to 0.8, more particularly 0.6 to 0.75 are observed.

In another aspect, the present description provides a device comprising an array of liquid crystalline polymer microneedles wherein the microneedles have a depth of penetration of from 50 to 120 microns using 8.89 Newton (2 pounds of force) for 10 seconds.

In another aspect, the present description provides a device comprising an array of liquid crystalline polymer microneedles wherein the microneedles have a depth of penetration of from 50 to 150 microns using 13.3 Newton (3 pounds of force) for10 seconds.

One of skill in the art will understand that depth of penetration studies may be influenced by the choice of substrate being penetrated. For instance, as discussed in more detail in the examples, depth of penetration study results on swine differ depending on whether they are conducted at the ham or the rib. In the present application, unless otherwise noted, depth of penetration values are provided as measured by application to the rib section of a swine.

In one further aspect, the present description provides device comprising an array of liquid crystalline polymer microneedles wherein the microneedles have a penetration efficiency using 13.3 Newton (3 pounds of force) for 10 seconds of 70% or higher, 80% or higher, or even 90% or higher. In this context, penetration efficiency refers to, for a given microneedle array, the ratio of microneedles that penetrate the skin to the total number of needles in the array. More particular description of the measurement technique is described in the Examples.

Again in this context, penetration efficiency studies may be influenced by the choice of substrate being penetrated. In the present application, unless otherwise noted, penetration efficiency values are provided as measured by application to the ham section of a swine.

In yet another aspect, the present description relates to hand insertable molded plastic microneedles. More specifically, the microneedles may be thermotropic liquid crystal polymer. In one embodiment, by hand insertable, it is meant insertable into human skin using normal hand applied pressures. For instance, suitable hand applied pressures may be less than 35.5 Newton (8 pounds of force), less than 22.4 Newton (5 pounds of force), or even less than 13.34 Newton (3 pounds of force).

It may be understood that testing such devices on humans may not be convenient, thus determining hand insertability may require the use of animal proxies. Those skilled in the art typically consider pig skin to be a close animal model of human skin. Thus, in one embodiment, the microneedles may be hand insertable into pig skin using normal hand applied pressures. For instance, suitable hand applied pressures may be less than 35.5 Newton (8 pounds of force), less than 22.4 Newton (5 pounds of force), or even less than 13.34 Newton (3 pounds of force).

In a further embodiments, the force required to insert devices of the present description into human skin may be low enough, and the microneedles may have a length that is small enough, that the hand insertion of such devices into human skin causes little or no bleeding. This is in contrast, for instance, to lancets, hypodermic needles, or the like, that, because of the penetration depth due to their length, typically cause bleeding when inserted into the skin.

Devices and microneedle arrays according to the present description may be used in a number of capacities. For instance, hollow microneedles or microneedle arrays may be used to deliver a formulation of active agent into the intradermal space. Further, hollow microneedles or microneedle arrays may be used to extract, using capillary force, suction, or other appropriate means, to extract fluids from skin (e.g., interstitial fluids, and the like). Solid microncedles or microneedle arrays according to the present description can be used in a number of ways. Solid microneedles or microneedle arrays may be coated with an active agent and used to deliver the active agent through the stratum corneum and into the intradermal space of the skin. Solid microneedles or microneedle arrays may also be used to penetrate the stratum corneum, providing channels into the intradermal space, which may be followed by a post treatment such as application of a transdermal patch, a cream, or the like. Further, channels may be made in the microneedles (for instance, with appropriately shaped mold cavities), which channels may be configured to allow for extraction of fluids (e.g., interstitial fluids) via capillary action.

The following Examples have been selected merely to further illustrate features, advantages, and other details of the invention. It is to be expressly understood, however, that while the Examples serve this purpose, the particulars of each Example are not to be construed in a manner that would unduly limit the scope of this invention.

### EXAMPLES

### Example 1

The depth of penetration (DOP) of liquid crystalline polymer microneedle arrays, applied in a manner to simulate hand application to skin, was determined in Yorkshire cross domestic pigs (Midwest Research Swine, Gibbon, Minnesota), *in vivo.* Each array was molded using Vectra MT1300 thermotropic liquid crystal polymer (Ticona Engineering Polymers, Florence, Kentucky) and featured four-sided pyramidal-shaped microneedles in a pattern of 15 rows x 15 columns with microneedle heights of nominally 700 microns, an aspect ratio of approximately 3.1 and tip-to-tip distance between neighboring microneedles of nominally 550 microns.

Prior to application, the microneedles were completely covered with a thin opaque coating of Rhodamine B for determination of the DOP. The arrays were primed using a two step priming process: Step 1) the arrays were flood coated with 35 µl of 0.5 mg/ml Poly-vinyl alcohol(80% hydrolyzed)(Sigma-Aldrich, Inc, St. Louis, Missouri) with 35 µg/ml Tween® 80 (Sigma-Aldrich, Inc, St. Louis, Missouri) in 90% (v/v) ethanol/water and dried at 35 °C (95 °F) for 20 minutes, and Step 2) the arrays were flood coated with 35 µl of an aqueous solution of 33.3 mg/ml Alum Potassium Sulfate (Penta Manufacturing, Livingston, New Jersey) and dried at 35 °C (95 °F) for 30 minutes. Primed arrays were then flood coated with 35 µl of an aqueous solution of 0.08% (w/v) Rhodamine B (Aldrich Chemical Company, Inc, Milwaukee, Wisconsin) and dried at 35 °C (95 °F) for 30 minutes.

Application sites on the animals were clipped then shaved as described in Example 2 to remove hair from the rib and ham areas, and the animals were anesthetized with isoflurane gas and maintained under anesthesia throughout the experiment.

Rhodamine B coated TLCP microneedle arrays were attached with double-sided pressure-sensitive tape to the shaft of a Chatillon® force gauge (model DFS-050) to monitor the level of application force. The arrays were applied to the pig skin using forces of 8.89 Newton (2 lbf), 13.34 Newton (3 lbf), and 22.24 Newton (5 lbf) for 10 seconds. Test arrays (n=3) were applied at each application force to both tissue over bone (rib area), and tissue over firm muscle (ham area).

The depth of penetration into the skin was determined indirectly by measuring the distance from the tip of the microneedle to where the Rhodamine B coating was wiped or dissolved from the microneedle after application into the skin. Analysis of the penetration depth was measured by imaging the microneedles using a microscope with Image Pro® Plus digital image analysis software (Media Cybernetics, Inc, Bethesda, Maryland). The mean DOP of each array was determined by measuring 66 of 225 total microneedles per array in four areas of the array pattern.

Depth of penetration results of Rhodamine B coated TLCP microneedle arrays that were applied to pig skin are presented in Table 1 for arrays applied to tissue over bone (rib area), and presented in Table 2 for arrays applied to tissue over firm muscle (ham area).

**Table 1: DOP vs Application Force applied to rib area of pig, in vivo.**

| Application Force | 8.89 Newton (2 lbf) | 13.34 Newton (3 lbf) | 22.24 Newton (5 lbf) |
|---|---|---|---|
| Mean DOP (n=3) | 85 µm | 100 µm | 138 µm |
| Standard Deviation | 4.6 µm | 18.0 µm | 12.7 µm |
| % RSD | 5.4% | 18.1% | 9.3% |

**Table 2: DOP vs Application Force applied to ham area of pig, in vivo.**

| Application Force | 8.89 Newton (2 lbf) | 13.34 Newton (3 lbf) | 22.24 Newton (5 lbf) |
|---|---|---|---|
| Mean DOP (n=3) | 70 µm | 82 µm | 94 µm |
| Standard Deviation | 22.6 µm | 11.7 µm | 3.4 µm |
| % RSD | 32.4% | 14.4% | 3.6% |

### Example 2

In a comparative study, microneedle arrays were molded using Vectra MT-1300 thermotropic liquid crystal polymer and arrays were also molded using LEXAN HPS1R-1125 polycarbonate (PC) (GE Plastics, Pittsfield, Massachusetts). The same mold insert was used in each case. The microneedle arrays featured three-sided pyramidal-shaped microneedles in a pattern of 14 rows x 14 columns with microneedle heights of nominally 700 microns and tip-to-tip distance between neighboring microneedles of nominally 500 microns. Individual needles were three-sided with one wall normal to the plane of the array base and the other 2 sloped. The vertical microneedle wall was 275 microns wide where it met the array base and the angles between the three walls of the needle were all 60 degrees.

The ability of the microneedles to penetrate skin with a simulated hand applied force was conducted using Yorkshire cross domestic pigs (Midwest Research Swine, Gibbon, Minnesota) *in vivo.* The ham area was first trimmed with an electric clipper (Oster Golden A5 clipper with a #50 blade) then shaved using a razor and a shaving cream. The ham was then rinsed with deionized water and wiped with 70/30 isopropanol water. A microneedle array was applied to the ham area and the applied force was monitored using a force meter (Ametek Mansfield & Green Division Accu Force Cadet with a range of 0-22.4 Newton (5 lbf)). The microneedle array to be tested was placed on the surface of the skin and then force was applied to the array using the force meter at the specified force for the specified period of time.

Penetration of the stratum corneum of the skin by the microneedles was assessed by *in vivo* staining post-application with a staining solution of Methylene Blue (2.0 mg/ml) containing Tween 80 (2.0 mg/ ml) in de-ionzed water. After application of a microneedle array, the array was removed from the skin and the application site was wetted with a cotton-tip applicator saturated with the methylene blue staining solution. The application site was immediately covered with a Hill Top Chamber® system, (Hill Top Research, Inc., Cinncinnati, Ohio) that was near saturation with methylene blue staining solution to maintain contact of the liquid with the skin. After 10 minutes staining time, the Hill Top Chamber® was removed, and the application site was rinsed well with de-ionized water, and dried by blotting with a paper towel. The site was then photographed.

Breaks in the stratum corneum layer as result of penetration of the microneedles allowed diffusion of the staining solution to the underlying tissue, resulting in blue stained holes. The pattern of stained holes, (i.e. spacing between needles and needle pattern of array) was visible, and easily discerned from hair follicles or areas of skin damage caused by scraping or scratches. The number of needle penetration sites was determined by counting the corresponding blue dots.

A total of six replicates were conducted using TLCP microneedle arrays applied with 13.34 Newton (3 lbf) for 10 seconds to the pig ham. A total of six replicates were conducted using PC microneedle arrays applied with 22.24 Newton (5 lbf) for 30 seconds to the pig ham. The results of the individual replicates for each array type were averaged and reported in the formal of percentage of microneedles in an array penetrating the application site (Table 3). An average of 89% of the TLCP microneedles in an array penetrated the pig ham, while an average of 36% of the PC microneedles in an array penetrated the pig ham.

**Table 3:**

| Description | TLCP Array 13.34 Newton (3 lbf) for 10 sec | PC Array 22.24 Newton (5 lbf) for 30 sec |
|---|---|---|
| | (n=6) | (n = 6) |
| Percentage of Microneedles in an Array Penetrating Pig Ham | 89% | 36% |
| Standard Deviation | 8.6% | 17.8% |
| %RSD | 9.7% | 49% |

### Example 3

The X-ray diffraction (XRD) analysis of the TLCP microneedles was conduced using the following procedure. The microneedle array was molded using Vectra MT1300 thermotropic liquid crystal polymer (Ticona Engineering Polymers, Florence, Kentucky). The TLCP microneedle array featured 316 four-sided pyramidal-shaped microneedles in an octagonal pattern, with microneedle heights of nominally 500 microns, an aspect ratio of approximately 3.1 and tip-to-tip distance between neighboring microneedles of nominally 550 microns. A row of microneedles was removed from the array by sectioning normal to the array base plane on both sides and parallel to the microneedle row. The resulting row of micro needles was mounted to a 1 mm flat glass strip with double coated adhesive tape. Five individual microneedles in the row were examined. The transmission geometry data was collected using a Bruker GADDS microdiffractometer (Bruker-AXS, Madison, Wisconsin), copper Kα radiation, and a VÅNTEC 2000 2D position sensitive detector (Bruker-AXS). The 500 µm pinhole collimated incident x-ray beam was conditioned by a graphite monochromator and directed normal to the sample needle axis with the needle positioned so that the long axis of the needle was placed in the vertical direction. Data were accumulated for 3 hours at a sample to detector distance of 12 cm. Detector electronics were set at 2048 x 2048 channels across the 2D detector registry plane and centered at 0 degrees (2Theta). A sample tilt angle (ω) of zero and X-ray generator settings of 50 kV and 50mA were employed. The observed 2D data was processed and corrected for spatial linearity and sensitivity using Bruker GADDS software (version 4.1.32, Bruker-AXS). Each 2D detector image was used to identify the diffraction peak maximum. Alignment of polymer chains with the microneedle long axis caused a maximum to occur along the equator of the 2D detector image. The TLCP microneedles produced a measured mean (n=5) equatorial diffraction peak maximum at a scattering angle of 19.7 degrees (2Theta) (standard deviation 0.1 degrees, % RSD 0.5%).

Azimuthal traces centered on the maxima having a 0.5 degree scattering angle (2Theta) window width over the 360 degrees of azimuthal angle were selected from the 2D data set using a 1 degree angular step size. Azimuthal trace data were inspected to identify angular positions of maxima in the data. The center of azimuthal maxima were identified and assigned an angular phi (ϕ) value of zero. Observed azimuthal intensity data between phi=0 and phi = 90 were tabulated. The azimuthal background intensity (I-bkg) value was evaluated as the mean azimuthal intensity at phi values between 85 and 90 degrees. The mean azimuthal background value (I-bkg) was subtracted from each observed azimuthal intensity data (1-observed) between phi = 0 and phi = 90 degrees to produce corrected intensity values (Iϕ). Hermans orientation function values (f) were determined using the procedure in the reference X-ray Diffraction Methods in Polymer Science, Leroy E. Alexander (1969), John Wiley & Son, Inc., New York; Chapter 4, "Preferred Orientation in Polymers". The calculations of Hermans orientation function and visualization of the data were conducted using the presentation and calculation software ORIGIN version 6.1 (MicroLab Corp., Northampton, Massachussetts). The mean (n=5) Hermans orientation function for the TLCP microneedles was 0.61 (Standard Deviation 0.12, %RSD 19.6%). The measured mean azimuthal peak width for the TLCP microneedles was 33.9 degrees (Standard Deviation 1.4 degrees, %RSD 4.1%).

### Example 4

The Buckle Force (P) of individual microneedles in an array was determined using a micromechanical tester (a modified version of the general design described in Figure 4 of the reference: Parker, E.R., Rao, M.P., Turner, K.L.,Meinhart, C.D., MacDonald, N.C., Journal of Micromechanical Systems, 2007, vol. 16, pp 289-295, the details of the modification are provided below). Microneedles were tested in an array that was molded using VectraMT1300 TLCP (Ticona Engineering Polymers, Florence, Kentucky). The TLCP microneedle array featured 316 four-sided pyramidal-shaped microneedles in an octagonal pattern, with microneedle heights of nominally 500 microns, an aspect ratio of approximately 3.1 and tip-to-tip distance between neighboring microneedles of nominally 550 microns.

The array was mounted with epoxy to a mechanical test bed so that the axial orientation of the microneedles was 0 degrees with respect to the long axis of the probe (i.e. tips of the microneedles oriented toward the probe). The test bed contained a manual z-axis micrometer control.

A National Jet NAJET Flat Nose Punch Series 100 (Material HSSC ; Shank 0.040", OAL 7/8". 0.002" x 7X) (The National Jet Company, LaVale, Maryland) was attached with epoxy to a threaded insert which was then attached to a GSO-150 load cell (Transducer Techniques, Temecula, California). The load cell was mounted on a TLSR-75B 2-axis motion stage (x-axis and y-axis control) that was positioned facing the mechanical test bed. The stage featured a programmable velocity ramp and a joystick drive (Zaber Technologies, Vancouver, Canada). The position gauge was a Lion CPL 290 Capacitance Gauge (Lion Precision, Saint Paul, MN). The advancement of the punch toward a microneedle was monitored using a VH-220 digital microscope having 200X magnification (Keyence Corporation, Osaka, Japan). Cursors on the microscope were used to align the punch so that the center of the punch impacted the tip of the microneedle. The punch was programmed to advance at a speed of 4 micrometers/second. The load displacement data was recorded in real time and analyzed using the graphical software package Igor Pro version 3.1.5 (WaveMetrics, Lake Oswego, Oregon). Recorded voltages for the force and displacement data were converted to units of force [N], and displacement [micrometers]. Force versus sample point was plotted and the maximum force applied before a sudden drop in force was observed in the plot was recorded as the Buckle Force (P). The mean (n=4) Buckle Force value for a single TLCP microneedle was measured as 0.34 N (Standard Deviation 0.03 N, %RSD 8.8%).

A comparator example was prepared in which the microneedle array was molded using LEXAN HPS1R-1125 polycarbonate (PC) (GE Plastics, Pittsfield, Massachusetts). The PC microneedle array featured 366 four-sided pyramidal-shaped microneedles in an octagonal pattern, with microneedle heights of nominally 500 microns, an aspect ratio of approximately 3.1 and tip-to-tip distance between neighboring microneedles of nominally 550 microns. The mean (n=3) Buckle Force value for a single PC microneedle was measured as 0.15 N (Standard Deviation 0.017 N, %RSD 11.3%).

### Example 5

The Maximum Sustained Bending Moment (M) of a single microneedle in a TLCP microneedle array was determined using the same array type, instrumentation, and assay conditions as in Example 4 with the following changes. The analysis of the data for bending moment assumes that a microneedle can be modeled as a simply supported cantilever. The array was mounted so that the axial orientation of the microneedles in the array was 90 degrees with respect to the long axis of the probe. In order to access interior microneedles, the array was sectioned normal to the array base plane. The punch was aligned so that the center of the punch impacted the microneedle at a position measured either 15% or 60% of the total length of the microneedle as measured from the tip to the base.

Depending upon the test, 15% or 60% of the microneedle height was calculated based on the initial height measurement of the microneedle. All measurements were referenced from the tip of the microneedle ( for example a deflection test at 15% first involved measuring the total length of the needle, calculating 15 percent of this value, then locating the probe down 15% of the length, measured from the tip of the microneedle). The probe was then positioned so that the center point of the punch was located on a centerline going through the microneedle normal to the microneedle's long axis at the location where the test was to be conducted. The lever arm in the determination of bending moment was taken to be; *lever* = L - L 15% , where L is the length of the needle, and L15% is 15% of the microneedle length. The Maximum Sustained Bending Moment (M) was calculated by multiplying the force maximum from the load-displacement with the length of the lever arm. Table 4 provides the mean Maximum Sustained Bending Moment (n=3) determined for a single TLCP microneedle in the array.

**Table 4: Maximum Sustained Bending Moment for a TLCP Microneedle**

| | TLCP Microneedle with the punch positioned 15% from tip of the Microneedle (mN-µm) | TLCP Microneedle with the punch positioned 60% from tip of the Microneedle (mN-µm) |
|---|---|---|
| Mean (n=3) | 43106 | 90351 |
| Standard Deviation | 4215 | 2650 |
| %RSD | 9.8% | 2.9% |

A comparator example was prepared in which the microneedle array was molded using LEXAN HPS1R-1125 polycarbonate (PC) (GE Plastics, Pittsfield, Massachusetts) The PC microneedle array was the same as in Example 4. The mean Maximum Sustained Bending Moment (n=3) determined for a single comparator PC microneedle in the array is presented in Table 5.

**Table 5: Maximum Sustained Bending Moment for a PC Microneedle**

| | PC Microneedle with the punch positioned 15% from tip of the Microneedle (mN-µm) | PC Microneedle with the punch positioned 60% from tip of the Microneedle (mN-µm) |
|---|---|---|
| Mean (n=3) | 29027 | 73618 |
| Standard Deviation | 1401 | 9297 |
| %RSD | 4.8% | 12.6% |

### Example 6

The elastic response of the microneedle is measured by E, the Elastic Modulus, while the plastic (resistance to permanent deformation) response is denoted by H, the hardness of the material. The nanoindentation measurement process was used to measure Elastic Modulus (E) and Hardness (H) as a continuous function of depth into the surface of the microneedle. Elastic Modulus and Hardness data were averaged about a spatial window of 1000 nm for a single point assessment of Hardness and Modulus. The TLCP microneedle array type and PC microneedle array type were the same as in Example 4.

The microneedles to be tested were cross-sectioned via microtoming after epoxy mounting at either the microneedle tip or 15% down the microneedle length measuring from the tip. An Agilent Nanoindenter XP (Agilent Technologies, Santa Clara, California) was used to examine a single microneedle of the array at the edge of microneedle and at the center of microneedle.

A diamond Berkovich probe was used and the spatial drift setpoint was set at 0.8 nm/s maximum. A constant strain rate experiment was run at 0.05 /s to a command depth of 1000 nm. The areas to be tested were located as seen top-down viewed through a video screen with 400X magnification. The test regions were selected locally with 400 X video magnification of the Nanoindentor XP to insure that tested regions were representative of the sample material (i.e. free of voids, inclusions, or debris). The dynamic excitation frequency and amplitude of the indenter was held constant at 45 hz and 1 nm for all experiments. Multiple tests were performed for each sample to assess reproducibility. Elastic Modulus and Hardness were determined via the Nanoindenter XP embedded CSM method *'XP CSM Standard Hardness Modulus and Tip Cal*' assuming a Poisson ratio of 0.35. Mean values for Elastic Modulus (E) and Hardness (H) are reported in the units of GPa and are presented in Tables 6-8.

**Table 6: Elastic Modulus (E) and Hardness (H) of TLCP Microneedles Measured at Tip**

| | Elastic Modulus at Edge (GPa) (n=1 ) | Elastic Modulus at Center (GPa) (n=3) | Hardness at Edge (GPa) (n=1) | Hardness at Center (GPa) (n=3) |
|---|---|---|---|---|
| Microneedle Tip (TLCP) | 6.3 | 4.7 | 0.26 | 0.16 |
| Standard Deviation | **** | 0.10 | **** | 0.02 |
| %RSD | **** | 2.2% | **** | 12.5% |

**Table 7: Elastic Modulus (E) and Hardness (H) of TLCP Microneedles Measured at 15% Distance from Tip**

| | Elastic Modulus at Edge (GPa) (n=3) | Elastic Modulus at Center(GPa) (n=3) | Hardness at Edge (GPa) (n=3) | Hardness at Center (GPa) (n=3) |
|---|---|---|---|---|
| Microneedle 15% from Tip | 7.3 | 4.4 | 0.24 | 0.15 |
| (TLCP) | | | | |
| Standard Deviation | 0.7 | 0.18 | 0.06 | 0.005 |
| %RSD | 9.6% | 4.0% | 25.0% | 3.3% |

**Table 8: Elastic Modulus (E) and Hardness (H) of PC Microneedles Measured at 15% Distance from Tip (Comparator Example)**

| Comparator Example | Elastic Modulus at Edge (GPa) (n=2) | Elastic Modulus at Center (GPa) (n=2) | Hardness at Edge (GPa) (n=2) | Hardness at Center (GPa) (n=2) |
|---|---|---|---|---|
| Microneedle 15% fromTip (PC) | 3.27 | 2.98 | 0.22 | 0.19 |
| Standard Deviation | 0.22 | 0.01 | 0.05 | 0.003 |
| %RSD | 6.6% | 0.33% | 22.7% | 1.5% |

## Claims

1. A device (10) comprising a thermotropic liquid crystalline polymer microneedle (100), wherein the microneedle (100) comprises a tip and a base, and wherein the microneedle (100) has a maximum sustained bending moment as measured at 15% of the distance from the tip to the base of from 30,000 to 60,000 mN-µm.

2. The device (10) of claim 1, wherein the microneedle (100) comprises mesogens that are molecularly aligned by an anisotropy factor in the range of from greater than 0.3 up to 1.0.

3. The device (10) of claim 1, further comprising a base, wherein the microneedle (100) is integral with and protrudes from the base, further wherein at least a portion of the mesogens are flow aligned.

4. The device (10) of claim 3, wherein at least about 30% of the mesogens are flow aligned.

5. The device (10) of claim 2, wherein at least about 10% of the mesogens are flow aligned, with the remainder of the mesogens having a relatively isotropic orientation state.

6. The device (10) of claim 1, wherein the microneedle (100) comprises a tip and a base, and wherein the microneedle has a maximum sustained bending moment as measured at 60% of the distance from the tip to the base of from 85,000 to 105,000 mN-µm.

7. The device (10) of claim 1, wherein the microneedle (100) has a buckling force of from 0.2 to 0.5 N.

8. The device (10) of claim 2, wherein the molecularly aligned mesogen-containing area of the microneedle (100) has an elastic modulus of from 6 to 8 Gpa.

9. The device (10) of claim 2 or 8, wherein the microneedle (100) Further comprises an area where the mesogens are substantially isotropic.

10. The device (10) of claim 9, wherein the isotropic area has an elastic modulus of from 4 to 6 Gpa.

11. The device (10) of claim 1, wherein the microneedle (100) has a Hermans orientation function value of from 0.4 to 0.8.

12. The device (10) of claim 1, wherein the thermotropic liquid crystalline polymer is a main chain thermotropic liquid crystalline polymer microneedle.

13. The device (10) of claim 1, wherein the thennotropic liquid crystalline polymer is a main chain polyester thermotropic liquid crystalline polymer microneedle.

## Patentansprüche

1. Vorrichtung (10), die eine Mikronadel (100) aus thermotropem, polymerem Flüssigkristall umfasst, wobei die Mikronadel (100) eine Spitze und eine Basis umfasst und wobei die Mikronadel (100) ein dauerhaftes maximales Biegemoment, gemessen bei 15 % des Abstands von der Spitze zur Basis, von 30.000 bis 60.000 mN-µm aufweist.

2. Vorrichtung (10) nach Anspruch 1, wobei die Mikronadel (100) Mesogene umfasst, die durch einen Anisotropiefaktor im Bereich von größer als 0,3 bis zu 1,0 molekular ausgerichtet sind.

3. Vorrichtung (10) nach Anspruch 1, die ferner eine Basis umfasst, wobei die Mikronadel (100) in die Basis eingebaut ist und aus dieser hervorragt, wobei ferner mindestens ein Teil der Mesogene strömungsausgerichtet sind.

4. Vorrichtung (10) nach Anspruch 3, wobei mindestens etwa 30 % der Mesogene strömungsausgerichtet sind.

5. Vorrichtung (10) nach Anspruch 2, wobei mindestens etwa 10 % der Mesogene strömungsausgerichtet sind, wobei die restlichen Mesogene einen relativ isotropen Ausrichtungszustand aufweisen.

6. Vorrichtung (10) nach Anspruch 1, wobei die Mikronadel (100) eine Spitze und eine Basis umfasst und wobei die Mikronadel ein dauerhaftes maximales Biegemoment, gemessen bei 60 % des Abstands von der Spitze zur Basis, von 85.000 bis 105.000 mN-µm aufweist.

7. Vorrichtung (10) nach Anspruch 1, wobei die Mikronadel (100) eine Knickkraft von 0,2 bis 0,5 N aufweist.

8. Vorrichtung (10) nach Anspruch 2, wobei der molekular ausgerichtete, die Mesogene enthaltende Bereich der Mikronadel (100) einen Elastizitätsmodul von 6 bis 8 GPa aufweist.

9. Vorrichtung (10) nach Anspruch 2 oder 8, wobei die Mikronadel (100) ferner einen Bereich umfasst, in dem die Mesogene im Wesentlichen isotrop sind.

10. Vorrichtung (10) nach Anspruch 9, wobei der isotrope Bereich einen Elastizitätsmodul von 4 bis 6 GPa aufweist.

11. Vorrichtung (10) nach Anspruch 1, wobei die Mikronadel (100) einen Hermanschen Orientierungsfunktionswert von 0,4 bis 0,8 aufweist.

12. Vorrichtung (10) nach Anspruch 1, wobei es sich bei dem thermotropen Flüssigkristallpolymer um eine Mikronadel aus thermotropem Hauptkettenflüssigkristallpolymer handelt.

13. Vorrichtung (10) nach Anspruch 1, wobei es sich bei dem thermotropen Flüssigkristallpolymer um eine Mikronadel aus thermotropem Hauptkettenpolyesterflüssigkristallpolymer handelt.

## Revendications

1. Dispositif (10) comprenant une micro-aiguille en polymère cristallin liquide thermotrope (100), dans lequel la micro-aiguille (100) comprend une extrémité et une base, et dans lequel la micro-aiguille (100) possède un moment maximal de flexion permanente tel que mesuré à 15 % de la distance de l'extrémité à la base allant de 30 000 à 60 000 mN-µm.

2. Dispositif (10) selon la revendication 1, dans lequel la micro-aiguille (100) comprend des mésogènes qui sont alignés du point de vue moléculaire par un facteur d'anisotropie dans la plage allant de plus de 0,3 jusqu'à 1,0.

3. Dispositif (10) selon la revendication 1, comprenant en outre une base, dans lequel la micro-aiguille (100) est solidaire et dépasse de la base, dans lequel en outre au moins une partie des mésogènes sont alignés avec l'écoulement.

4. Dispositif (10) selon la revendication 3, dans lequel au moins environ 30 % des mésogènes sont alignés avec l'écoulement.

5. Dispositif (10) selon la revendication 2, dans lequel au moins environ 10 % des mésogènes sont alignés avec l'écoulement, le reste des mésogènes possédant un état d'orientation relativement isotrope.

6. Dispositif (10) selon la revendication 1, dans lequel la micro-aiguille (100) comprend une extrémité et une base, et dans lequel la micro-aiguille comprend une extrémité et une base, et dans lequel la micro-aiguille (100) possède un moment maximal de flexion permanente tel que mesuré à 60 % de la distance de l'extrémité à la base allant de 85 000 à 105 000 mN-µm.

7. Dispositif (10) selon la revendication 1, dans lequel la micro-aiguille (100) a une force de flambage allant de 0,2 à 0,5 N.

8. Dispositif (10) selon la revendication 2, dans lequel la zone contenant des mésogènes alignés du point de vue moléculaire de la micro-aiguille (100) a un module élastique allant de 6 à 8 GPa.

9. Dispositif (10) selon la revendication 2 ou 8, dans lequel la micro-aiguille (100) comprend en outre une zone où les mésogènes sont essentiellement isotropes.

10. Dispositif (10) selon la revendication 9, dans lequel la zone isotrope a un module élastique allant de 4 à 6 GPa.

11. Dispositif (10) selon la revendication 1, dans lequel la micro-aiguille (100) a une valeur de fonction d'orientation de Hermans allant de 0,4 à 0,8.

12. Dispositif (10) selon la revendication 1, dans lequel le polymère cristallin liquide thermotrope est une micro-aiguille de polymère cristallin liquide thermotrope de chaîne principale.

13. Dispositif (10) selon la revendication 1, dans lequel le polymère cristallin liquide thermotrope est une micro-aiguille de polymère cristallin liquide thermotrope polyester de chaîne principale.
